(19) Europäisches Patentamt
European Patent Office
Office européen des brevets

(11) **EP 4 103 042 B1**

(12) **EUROPEAN PATENT SPECIFICATION**

(45) Date of publication and mention
of the grant of the patent:
**18.03.2026  Bulletin 2026/12**

(21) Application number: **21705642.3**

(22) Date of filing: **15.02.2021**

(51) International Patent Classification (IPC):
*A61F 5/56* (2006.01)  *A61B 5/00* (2006.01)
*A61M 21/00* (2006.01)  *A61B 5/0205* (2006.01)
*A61B 5/024* (2006.01)  *A61B 5/053* (2021.01)
*A61B 5/08* (2006.01)  *A61B 5/085* (2006.01)
*A61B 5/1455* (2006.01)  *A61H 9/00* (2006.01)
*A61H 23/02* (2006.01)  *A61M 16/00* (2006.01)
*H04R 1/34* (2006.01)  *H04R 9/06* (2006.01)
*A61H 23/00* (2006.01)

(52) Cooperative Patent Classification (CPC):
(C-Sets available)
**A61F 5/56; A61B 5/4818; A61H 9/0078;
A61H 9/0085; A61H 23/006; A61H 23/0218;
A61H 23/0236; A61H 23/0245; A61H 23/0263;
A61M 21/00;** A61B 5/0205; A61B 5/024;
A61B 5/053; A61B 5/0816; A61B 5/085;      (Cont.)

(86) International application number:
**PCT/NL2021/050098**

(87) International publication number:
**WO 2021/162555 (19.08.2021 Gazette 2021/33)**

(54) **APPARATUS FOR PREVENTION OF APNEA**

VORRICHTUNG ZUR VORBEUGUNG VON APNOE

APPAREIL POUR LA PRÉVENTION DE L'APNÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **14.02.2020  NL 2024918**

(43) Date of publication of application:
**21.12.2022  Bulletin 2022/51**

(73) Proprietor: **Academisch Ziekenhuis Leiden
(h.o.d.n. LUMC)
2333 ZA Leiden (NL)**

(72) Inventors:
• **TE PAS, Arjan Benedictus
2333 ZA Leiden (NL)**
• **CRAMER, Sophie Josephine Elisabeth
2300 ZA Leiden (NL)**
• **HOOPER, Stuart Brian
Clayton
Victoria 3168 (AU)**

(74) Representative: **HGF
HGF BV
Benoordenhoutseweg 46
2596 BC The Hague (NL)**

(56) References cited:
WO-A1-2015/175673      WO-A1-2019/036408
US-A1- 2010 318 007      US-A1- 2018 160 967

**EP 4 103 042 B1**

(52) Cooperative Patent Classification (CPC): (Cont.)
A61B 5/14551; A61B 5/271; A61B 5/318;
A61B 5/363; A61B 5/389; A61B 2503/045;
A61H 2023/002; A61H 2201/0146;
A61H 2201/0157; A61H 2201/1619;
A61H 2201/165; A61H 2201/1654;
A61H 2201/5007; A61H 2201/5028;
A61H 2201/5071; A61H 2203/0443; A61H 2230/04;
A61H 2230/045; A61H 2230/065; A61H 2230/206;
A61H 2230/208; A61H 2230/40; A61H 2230/405;
A61H 2230/425; A61M 2016/0015;
A61M 2021/0022; A61M 2205/0211;
A61M 2205/0238; A61M 2205/0294;
A61M 2205/103; A61M 2205/106; A61M 2205/18;
A61M 2205/3306; A61M 2205/3584;

A61M 2205/3592; A61M 2205/52; A61M 2205/583;
A61M 2205/8262; A61M 2209/088; A61M 2210/04;
A61M 2230/04; A61M 2230/06; A61M 2230/08;
A61M 2230/205; A61M 2230/40; A61M 2230/60;
A61M 2230/63; A61M 2230/65; A61M 2240/00;
H04R 1/345; H04R 9/06

C-Sets
A61M 2230/04, A61M 2230/005;
A61M 2230/06, A61M 2230/005;
A61M 2230/08, A61M 2230/005;
A61M 2230/205, A61M 2230/005;
A61M 2230/40, A61M 2230/005;
A61M 2230/60, A61M 2230/005;
A61M 2230/65, A61M 2230/005

**Description**

Field of the Invention

The present invention relates to medical devices generally, and particularly to medical devices for prevention of apnea. More particularly, this invention relates to medical devices for prevention and/or termination of apnea, hypoxia and bradycardia in preterm infants that impose low impact to their bodies.

Background of the Invention

Apnea of Prematurity (AOP) is one of the most common diagnosed ailments occurring in preterm infants, generally starting within the first week after birth and resolving with maturation. The incidence of AoP is inversely related to gestational age: 20% of infants born at 34 weeks' gestation, 85% of infants born at 30 weeks of gestation and essentially all infants born at ≤28 weeks' gestation are diagnosed with AoP. Severe and recurrent apneas are associated with cerebral injury and adverse neurodevelopmental outcome. Despite pharmacotherapy and respiratory support to prevent apneas, a proportion of infants continue to have apneas and often need tactile stimulation, mask, and bag ventilation and/or extra oxygen. The duration of the apnea and the concomitant hypoxia and bradycardia often depends on the response time of the nurse.

Various stimulation techniques have been shown to permit termination of apnea. E.g. manual and mechanical vibratory stimulation are equally effective. Automated stimulation, using closed-loop pulsating or vibrating systems, has been shown to be able to terminate apneas, but data is scarce. Several studies used continuous mechanical stimulation, with pulsating, vibrating, or oscillating stimuli, to prevent apneas, but the reported effect varied. A single publication discloses that pulsation significantly reduced central and mixed apnea. Different forms of vibrating stimuli were shown to significantly reduce apnea, hypoxia and bradycardia. Six studies used an oscillating mattress, but this form of stimulation did not lead to a consistent effect in reducing apnea. The effectiveness of (manual) tactile stimulation on the termination of apnea has however not been disclosed.

US-B-8517981 describes a method and apparatus for prevention of apnea of patients having episodes of postoperative respiratory depression. The method described comprises monitoring by an apparatus an individual's oxygen saturation and/or respiration, coupled to a stimulus delivery device, which stimulus delivery device can comprise audible stimuli, tactile stimuli, and the administration of one or more doses of a narcotic antagonist.

In most Neonatal Intensive Care Units (NICUs) both pharmacotherapy and breathing support are used to prevent recurrent AOP. Despite these preventative interventions, a proportion of infants continue to have apnea, which requires further intervention of the caregiver. The termination of apnea is accomplished by tactile intervention of the nurse, often combined with extra oxygen and, if needed, mask ventilation. The duration of the apnea and the concomitant hypoxia and/or bradycardia is then dependent on the response time of the nurse. Heavy workload and alarm fatigue might have a negative influence on prompt and adequate treatment of apneas. The longer the delay in response time, the longer the total duration of apnea and the lower the peripheral oxygen saturation values.

Mechanical stimulation might improve the commonly used and effective tactile stimulation technique by enabling a direct response, as this will shorten the apnea hence reducing hypoxia and bradycardia. In addition, combining mechanical stimulation with the detection of imminent apnea could lead to preventive stimulation methods. The effect of mechanical tactile stimulation on apnea has been studied but has not led to implementation in the NICU or a commercially available device yet.

The magnitude of these challenges has increased with the increased survival of extremely low birth weight infants and the advent of single-family room care. There is strong endorsement by neonatologists for developing new approaches to stabilize breathing patterns and to prevent intermittent hypoxia and bradycardia episodes in preterm infants. Improvements in the current treatment will not only reduce the heavy burden faced by many patients and parents but also considerably decrease the economic burden of premature birth on our society.

US 2018160967 A1 describes a system for dealing with breathing anomalies, including apnea that can monitor a neonatal subject for indications of breathing anomalies while the subject is sleeping. Stimulating a volar surface by, for example a stroking motion via stroking actuators, or emulating such a movement by sequentially activating a linear array of actuators, can manipulate the subject's breathing. The device is comparatively complex, and the stimulation requires an actual stroking actuator, which may not work if not entirely on close contact with the subject's skin, and needs moving parts; or a relatively complex array of actuators that require handling. Yet further, there is no method disclosed that may lead to a guaranteed stroking sensation, which required adaptation for each subject. This may in particular be unsuitable for an infant, let alone a preterm infant, where the response may not be communicated well enough.

WO 2019036408 A1 describes a system for providing a stochastic mechanical stimulus via a mattress to patients, in particular preterm infants suffering from e.g. apnea, to improve their respiration. A stimulus disclosed as 'suitable' is applied after measurement and machine learning analysis of impaired respiration parameters. Again, there is no method disclosed that may lead to a guaranteed stroking sensation, which required adaptation for each subject. This may in particular be unsuitable for an infant, let alone a preterm infant, where the response may not be communicated well enough to lead to a guaranteed induced response.

US 2010318007 A1 describes an external electromechanical tactile stimulation device to be positioned against a person's lower spine, which comprises randomly actuated vibrators, for sleep enhancement (e.g. treating apnea). This may not be useful for an infant.

WO 2015175673 A1 describes a system and method for reducing apnea in a patient, comprising sensors for detecting the sleep state, software to determine the sleep state, and vibratory actuators to transmit vibration to the patient to change the sleep state. The vibration signal may be varied over time to reduce adaptation by the patient. However, this does not induce the stroking sensation which is particularly suitable for reduction of AOP.

Accordingly, there remains the need for an improved automated mechanical stimulation device for preterm infants that will shorten or prevent apnea even more by enabling a prompt response.

Summary of the Invention

It is therefore an object of the present invention to provide a device that will shorten or prevent apnea even more by enabling a prompt response. It is another object of the present invention to decrease the occurrence and/or shortening the duration of hypoxia and/or bradycardia, resulting in an improved neurodevelopmental outcome. It is a further object of the present invention to provide a device that will result in more accurate monitoring of the patient's condition. It is a further object of the invention to reduce the work load of the nurses and reduce the amount of alarms, to reduce the chance of cross-contamination and to make earlier discharge possible by providing an intervention that also can be used at home.

These and other objects are addressed by the device of the present invention. Accordingly, the present invention relates to an automated stimulation device for inducing a tactile inter-stimulus onset asynchrony (ISOA) effect in a subject suffering from apnea, bradycardia and/or hypoxia, the device comprising at least two actuators (2) configured for contacting a body portion of the subject, and interspaced for producing an apparent tactile movement in the subject upon sequential induction of actuation, characterized in that a duration of the actuations and an overlap in actuation time between the at least two actuators is controlled to attain an inter stimulus onset asynchrony (ISOA), wherein the Stimulus onset interval and duration are determined and controlled based on the following formula (I):

$$\textit{Stimulus onset interval (sec)} = 0.31 \textit{ x duration of stimulation (sec)} + 0.0473 \textit{ (I)}.$$

The present invention furthermore relates to a method for the treatment of apnea, bradycardia and/or oxygen desaturation in a subject, comprising:

a) providing an automated stimulation device for inducing a tactile inter-stimulus onset asynchrony (ISOA) effect in a subject suffering from apnoea, bradycardia and/or hypoxia, the device comprising at least two actuators configured for contacting a body portion of the subject, and interspaced for producing an apparent tactile movement in the subject upon sequential induction of actuation, wherein the duration of the actuations and the overlap in actuation time between the at least two actuators is controlled to attain an inter stimulus onset asynchrony (ISOA) in contact with a subject's body portion, and
b) monitoring the subject's blood oxygen saturation and/or respiration pattern and/or heart rate, and
c) delivering by the at least one stimulus delivery device a stimulus to the subject.

Short Description of the Figures

The present invention now is described more fully hereinafter with reference to the accompanying drawings, in which a preferred embodiment of the invention is shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

Figure 1 represents an automated stimulation device for inducing a tactile inter-stimulus onset asynchrony (ISOA) effect in a subject suffering from apnoea, bradycardia and/or oxygen desaturation, the device comprising two actuators with a maximum distance between the actuators.
Figure 2 represents possible locations on an infant where the automated stimulation device can be placed.
Figure 3 represents a schematic representation of the test set-up for testing the animals in the Biomedical Imaging Centre at the SPring-8 synchrotron in Japan.

Figure 4 represents a different test set-up, for testing the effect of the stimuli.

Figure 5 represents the effect of stimulation periods on heart rate and breathing rate per stimulation indicator as described in Example 3.

Figure 6 represents a top/frontal view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators.

Figure 7 represents a bottom view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators.

Figure 8A represents a side view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators.

Figure 8B represents a cross sectional side view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators. Figure 8B illustrates a section taken along the line B-B of Figure 7.

Figure 9 represents a flowchart representing a system configured to control and/or to provide feedback to an automated stimulation device for inducing a tactile inter-stimulus onset asynchrony (ISOA) effect in a subject suffering from apnoea, bradycardia and/or oxygen desaturation.

Figure 10 represents a flowchart representing an automated stimulation device for inducing a tactile inter-stimulus onset asynchrony (ISOA) effect in a subject suffering from apnoea, bradycardia and/or oxygen desaturation configured to be connected to two or more actuators.

Detailed Description of the invention

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

The term "apnea" as used herein, includes a cessation of breathing for 20 seconds or a shorter pause accompanied by bradycardia, cyanosis, or pallor. Based on their origin, apneic spells are classified as central, obstructive, or mixed. Central apnea is distinguished by a cessation of airflow due to absence of respiratory drive, obstructive apnea is characterized by impeded airflow caused by closure of the upper airways and mixed apnea implies that central respiratory pauses are followed by obstruction in the upper airways or vice versa. Most of the apneas in a preterm infant have a mixed character, starting with a central or an obstructive episode.

The term "inter stimulus onset asynchrony (ISOA)" or 'stimulus onset asynchrony (SOA) as used herein, includes a ratio described in the literature between the duration of stimuli and the delay between the onset of the stimuli. A stimulus may consist of, e.g., a sound, a tactile action, or a vibration. By choosing the right ratio between stimuli duration and the interval between the onset of the stimuli, an illusionary motion of the stimulus from the first stimulation point to the following stimulation points is sensed. A short time interval between the stimuli may lead to interference in the neural processing of these two patterns, thereby inducing a desired response.

The term "Pulse Width Modulation (PWM)" as used herein, includes a technique that controls the stimulation by creating a square wave by switching the signal of the frequency on and off (and thus the amplitude and strength). This on-off pattern can simulate voltages in between full on (for example PMW 255, 5V) and off (for example PMW 0, 0V) by changing the amount of time the signal is on versus off.

The term "preterm infant" as used herein, includes human infants (babies) born alive before 37 weeks of pregnancy are completed. There are sub-categories of preterm birth, based on gestational age: extremely preterm (less than 28 weeks), very preterm (28 to 32 weeks) and moderate to late preterm (32 to 37 weeks).

The present invention relates to an automatic tactile stimulation device for inducing a tactile inter stimulus onset asynchrony (ISOA) effect in a subject. This subject can be any animal or human being, it is preferably an infant, preterm or term, more preferably an infant suffering from apnea. Preterm infants often have apnea, and the more preterm they are, the more often it occurs. The device of the invention provides in shortening or preventing apnea more by enabling a prompt response.

The device comprises at least two actuators configured for, and arranged to producing a tactile sensation, more particularly an apparent tactile movement by controlling the stimulus duration of actuation and the overlap in actuation time between at least two actuators in order to attain an inter stimulus onset asynchrony (ISOA), which actuators further comprise a sensing system for detecting motion. The actuators are preferably in contact with the subjects' skin.

The number of actuators can vary, and is at least two, to generate a moving tactile sensation. With three actuators one can make a longer stroke, or a curved stroke, or a circle, depending on the use of a sequence of discrete tactile pulses of the same duration or of an algorithm that is being used. Furthermore, it can be designed that a single continuous moving stroke is being felt, or for example two strokes or even multiple strokes. For the purpose of this invention it is preferred to use 2 actuators. With 2 actuators the least space is being used, while still being able to prevent or shorten apnea. It is furthermore preferred to vibrate the actuators such, that a single stroke is being felt.

Preferably, the actuators are chosen from the group of unbalanced DC-motors, linear resonant actuators (LRA), voice coils or (loud)speakers (VC), solenoids and piezoceramic elements (PCE) or pneumatic balloon actuators, or any combination thereof.

Unbalanced Direct Current (DC) motors or eccentric rotating mass (ERM) vibration motors make use of electromagnetism to rotate an inner shaft. The fixation of an off-centre weight at the end of the shaft leads to vibration. The speed of the motor is affected by the input voltage. Only the frequency can be controlled in these motors. A higher input voltage results in a higher motor speed and therefore an increased vibration frequency and stroke. Unbalanced DC-motors are available in two types; the eccentric rotating mass or pager motor and the coin or pancake motor.

Loudspeaker or voice coil (VC) actuators typically comprise of a fixed magnet surrounded by a coil with a cone attached. When alternating current passes through the coil the magnetic field will alternately attract and repel the magnet. This means that the magnet will vibrate in the same frequency as the incoming voltage. The cone amplifies the sound of the vibration, by increasing the amount of air that is being moved. The stroke of a loudspeaker can be controlled independently from the frequency of the vibration by adjusting voltage. The resonance frequency depends on various characteristics and differs per loudspeaker. Bass speakers often have lower resonance frequencies compared to speaker that reproduces higher frequencies.

Linear resonant motors work in the same manner as a loud speaker. In the case of a linear resonant actuator (LRA), the change in magnetic field typically drives a mass suspended on a spring instead of a magnet. The movement of the mass causes vibrations. Also in LRA's the stroke and frequency can be modelled separately. However, unlike a speaker, this actuator is often only used for frequencies around its resonant frequency, where the LRA is most efficient in its operation. LRA's are able to operate at lower frequencies, but with a reduced stroke. The resonant frequency of small LRA's lies typically between 175 and 250 Hz.

A solenoid typically comprises of a coil which produces a magnetic field when electric current is passed through it. This actuator is equipped with a magnetic rod in the centre of the coil. Two types of solenoids exists; a push- and a pull-version. Activation of the magnetic field either pulls the rod in or pushes it out. When the voltage is switched off, the rod will move back by means of a spring or gravitation. A solenoid has a fixed stroke and the frequency is dependent of the on-off cycle. For AC solenoids, the maximum permissible temperature, caused by high cycling frequencies will be the limiting factor for the maximum switching frequency. DC solenoids will be restricted by the pull-in and drop-out times.

The piëzo-electric effect is a phenomenon in which certain materials changes shape when electrical voltage is applied. As this effect is reversible, a piëzo can serve as an actuator as well as a sensor. Piëzo elements are commercially available in a circular and rectangular shape. Although they have a very fast response, the disadvantage of piëzo elements is the relative high driving voltage (from 30Vp-p) that is required for small displacements (0.01-2 mm).

A pneumatic balloon actuator (PBA) comprises preferably a cavity, of which at least one side is made of flexible material, and one or more pneumatic supply channels. As pressure is applied through these channels, the PBA swells, and thus actuates. Pressure can be applied with solenoid valves, speakers or other moving and pressure changing elements. The cavity and supply channels can be filled with a fluid, such as a liquid fluid, or a gaseous fluid, such as air.

Advantageously, the actuators are pneumatic inflatable actuators for providing a direct tactile stimulus to a subjects' skin. Most preferred is to use pneumatic balloon actuators for providing a tactile stimulus to a subjects' skin. The advantage is that these pneumatic balloon actuators are light, easy to clean, and easy to apply to a subject's skin, and that they are soft, reducing the issue chance of harming the skin. In combination with the preferred speakers and/or solenoids to provide the pneumatic balloons with a pulse, the device is operable in a wide range of frequencies and amplitudes and requires no electrical wires or electrical elements within the incubator, as speaker/solenoid can be placed outside the incubator. Accordingly, int this preferred embodiment, only the pneumatic supply channels and the balloons are inside of an incubator, and in the vicinity of the subject, preferably an infant.

The actuators are advantageously shielded from the subject within a passive housing (also referred to as casing or shielding herein). This passive housing can be made of various materials. The passive housing is usually in a fixed configuration, and hence not moving. Thus, there are two options: either the actuator will vibrate as a whole on the skin and uses inertia to deform the skin or the actuator can push itself away from passive housing, which provides a counterforce and anchoring point. The housing may comprise a mattress, but it might also be a shielding or casing around the actuator, when placed on top of the subject, that will provide counterforce by its gravity, and hence of sufficient weight. Other options for achieving a good contact with the skin of a subject and a transmission of the movement include straps, or clothes or garments around a member or portion of the subject that keep the shielding in its place, or a shield that is attached by suction, griping or clamping to the skin of the subject.

A preferred material for the casing of the actuator, or the actuator itself if directly in contact with the subject comprises a silicone polymer, more preferred a medical grade silicone. Medical grade silicone are silicones tested for biocompatibility and appropriate to be used for medical applications. Even more preferred is to use inflatable actuators, preferably pneumatic balloon actuators made of medical grade silicone.

The actuators can be adhered to the subject in various ways, as long as the subject still feels the vibrations. Options are to incorporate the actuators in a mattress, or to incorporate it into straps, or in garments clothes, for example in a baby bodysuit, (cotton) napkins, or in other underwear or T-shirt. Another option is to attach the actuators to a diaper, or to wrap it around the body with a Velcro closure.

In a particularly preferred embodiment, the pneumatic balloon actuators are composed essentially of medical grade silicone, as this permits to place the actuator balloons directly into contact with the skin of the subject, and they preferably automatically stay in place, as the silicone materials have a high friction coefficient. This is particularly effective in case the subject is a preterm infant, as such infants usually do not move their trunk.

An advantage of incorporation of the actuators in a mattress is that there is no additional or extra device present or required in the incubator. An advantage of placing the actuators directly on the skin of the subject is that the location can be chosen by the nurse, in particular in the case of pressure marks or damaged skin, and the strength of the stimulus is not dependent on the weight of the infant.

To clarify, in order to attain the sensation of a tactile stroke by an inter stimulus onset asynchrony (ISOA), two stimuli are coordinated to be applied within a Stimulus onset interval. Accordingly, the duration of the actuations and the overlap in actuation time between the at least two actuators is controlled to attain an inter stimulus onset asynchrony (ISOA). To clarify, ISOA is attained when (at least) two stimuli are initiated at different starting times and the delay between the onset of the stimuli is controlled along with the duration of the stimuli.

The actuators of the automatic tactile stimulation device are stimulated, with a stimulus onset interval and duration that are determined based on the following formula (I):

$$\textit{Stimulus onset interval (sec)} = 0.31 \times \textit{duration of stim (sec)} + 0.0473 \qquad (I)$$

whereby preferably the duration of the stimulation is in the range of from 0.5 up to 5 seconds, more preferably in the range of from 1 up to 3 seconds, as c-tactile afferents, the receptors that respond to stroking, are most sensitive for a dynamic stimulus of 1 to 10 cm/sec.

The actuators of the automatic tactile stimulation device are preferably stimulated at a vibration frequency in the range of from 2 or 5 up to 350 Hz, more preferably in the range of from 2 or 10 up to 200 Hz, even more preferably in the range of from 2 or 10 up to 100 Hz, of from 30 up to 60 Hz, or of from 2 up to 10 Hz.

The actuators of the automatic tactile stimulation device are preferably spaced at a distance of from 2 to 20 cm from each other, more preferably at a distance of from 4 up to 15 cm. The distance can be flexible when attached to straps of applied loose on the skin (with silicon), and fixed when the actuators are integrated into the clothes or mattress. The distance for extreme preterm infants will preferably be in the range of from 5 cm up to 10 cm and for term infants preferably of from 10 cm up to 20 cm.

The automatic tactile stimulation device preferably comprises a sensing system for detecting apnea/bradycardia/desaturation. This sensing system is preferably one or more sensors for monitoring the subject's oxygen saturation and/or respiration and/or heart rate, more preferably wherein the device comprises a pulse oximeter and/or respiratory monitor, and/or ECG coupled to at least one actuator. The subject's oxygen saturation and/or respiration are preferably measured using ECG, EMG and/or thoracic impedance. Thus, preferably the automatic tactile stimulation device further comprising one or more sensors for monitoring the subject's oxygen saturation, respiration and/or heart rate, more preferably wherein the device comprises a pulse oximeter, respiratory monitor and/or ECG, even more preferably wherein the one or more sensors are coupled to, or integral with at least one actuator.

The automatic tactile stimulation device preferably comprises a computer-executable algorithm for predicting apnea, bradycardia or desaturation. The algorithm can be stored on a non-transitory computer-readable medium and can be executed by a processor. The algorithm can be based on historical data of an individual subject or on reference data of a cohort of subjects similar to the subject. The data comprises information gathered by the sensing system. The algorithm can be generated or adjusted via a machine-learning or artificial intelligence-based algorithm based on data gathered by the sensing system so that a bespoke algorithm can be produced for each individual subject. The algorithm for predicting apnea, bradycardia or desaturation can be used, when apnea, bradycardia or desaturation is predicted, by a processor to stimulate the actuators of the automatic tactile stimulation device.

In a preferred embodiment the stimulus is provided by at least two inflatable actuators in fluid connection to two independently controllable solenoid valves or speakers, more preferably further comprising a source of air pressure coupled to the valves. Thus, the principle of the preferred pneumatic balloon actuator (PBA) with the design of a tactor is combined, resulting in two balloons that are made of medical grade silicone are airtightly connected to two separate controllable solenoid valves or speakers. The solenoid valves or speakers will be placed outside the incubator. The advantage of this combination is that the frequency can be adjusted in a very wide range, without having to place a very large or hot actuator on the skin of the infant. Furthermore, AC driven actuators are able to generate stochastic stimulation, which is expected to prevent habituation. In order to transmit the vibration of the actuator to the balloon with the least force, the air tubes are preferably as stiff and short as possible. The actuators are preferably between 3 and 7 mm diameter, made of medical grade silicon. The supply channels are preferably between 30 and 50 cm, with an outer diameter of preferably between 3 and 7 mm.

The inflatable actuators, preferably balloons, are preferably shielded with a silicon strap that may follow the shape of the infant's body. The advantage is that the strap can easily be cleaned or be manufactured as a disposable. As the silicon is light but has a high friction coefficient, the strap will stay in place without applying a lot of weight on the vulnerable skin. An additional advantage of this preferred embodiment is that the balloons can also be used to detect breathing, as breathing might compress the balloons leading to a peak in pressure. A pressure sensing system is then provided for, preferably incorporated within the actuator.

The present invention furthermore directed to a method for the treatment or prevention of apnea, bradycardia and/or oxygen desaturation (hypoxia) in a subject, comprising:

a) providing an automated stimulation device for inducing a tactile inter-stimulus onset asynchrony (ISOA) effect in a subject suffering from apnoea, bradycardia and/or hypoxia, the device comprising at least two actuators configured for contacting a body portion of the subject, and interspaced for producing an apparent tactile movement in the subject upon sequential induction of actuation, wherein the duration of the actuations and the overlap in actuation time between the at least two actuators is controlled to attain an inter stimulus onset asynchrony (ISOA) in contact with a subject's body portion, and
b) monitoring the subject's blood oxygen saturation and/or respiration pattern and/or heart rate, and
c) delivering at least one stimulus to the subject by the stimulation delivery device.

Delivering at least one stimulus can be done in regular intervals, such as every 5 or 10 minutes, or irregular randomly chosen intervals, in order to reduce the amount of apnea, desaturation and bradycardia.

In step b) of the method, monitoring the subject's blood oxygen saturation and/or respiration pattern and/or heart rate preferably comprises using a pulse oximeter and/or respiratory monitor and/or heart rate in contact with the subject, more preferably wherein a pulse oximeter and/or respiratory monitor/ECG is coupled to the at least one stimulus delivery device.

Alternatively, or in addition to, step b) of the method comprises using a computer-executable algorithm for predicting apnea, bradycardia or desaturation. The algorithm can be executed by a processor. The algorithm can be based on historical data of an individual subject or on reference data of a cohort of subjects similar to the subject. The data comprises information gathered by the sensing system. The algorithm can be generated or adjusted via a machine-learning or artificial intelligence-based algorithm based on data gathered by the sensing system so that a bespoke algorithm can be produced for each individual subject. The algorithm for predicting apnea, bradycardia or desaturation can be used, when apnea, bradycardia or desaturation is predicted, by a processor to decide whether or not to deliver a stimulus according to step c) of the method.

The stimulus of step c) is preferably administered when the subject's oxygen saturation and/or respiration falls within a first predefined range and/or is sustained within the first predefined range for a first predefined duration, e.g. indicating a breathing anomaly. Alternatively, stimulation may also be applied following a predictive model, and thereby not necessarily rely on a feedback mechanism involving analysis of the breathing behaviour.

In step c) of the method, preferably the tactile inter stimulus onset asynchrony (ISOA) is used to induce the sensation of a moving stimulus, thereby stimulating a large area to effect a desired response in the subject, and inducing a reflex reaction, in particular a Babinski reflex, which is one of the normal reflexes in infants. Such reflexes are responses that occur when the body receives a certain stimulus, whereby the Babinski reflex occurs once the sole of the foot is firmly stroked. Applicants believe that a response that alters the breathing behaviour can also be induced into subjects, in particular infants, and pre-term infants, when applying a tactile inter-stimulus onset asynchrony (ISOA) effect, in particular one that is usually associated with a stroking sensation, to the skin, and not necessarily limited only to volar skin of the foot or hand. Accordingly, the subject to apply the method on preferably may be a premature or a term infant.

The present invention now is described more fully hereinafter with reference to the accompanying drawings, in which preferred embodiments of the invention are shown.

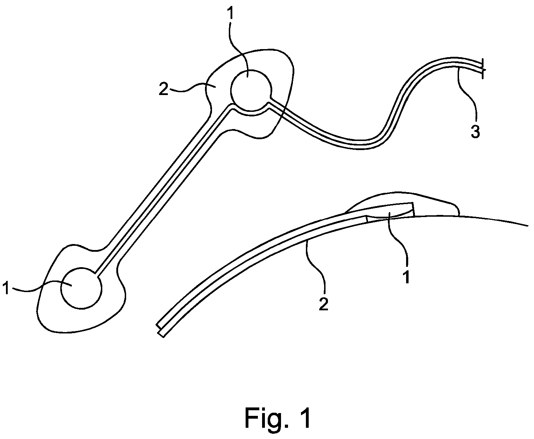

Figure 1 represents an automated stimulation device for inducing a tactile inter-stimulus onset asynchrony (ISOA) effect in a subject suffering from apnoea, bradycardia and/or oxygen desaturation, the device comprising two actuators with a maximum distance between the actuators. Two balloons (1) that are made of medical grade silicone and are surrounded by a silicone shield (2) are airtight connected via air tubes (3) to two separate controllable solenoid valves or speakers, or otherwise devices that induce a vibration. The silicon shield will follow the shape of the body. The solenoid valves or speakers may preferably be placed outside the incubator. The advantage of this combination is that the frequency can be adjusted in a very wide range, without having to place a very large or hot actuator on the skin of the infant.

Furthermore, actuators, in particular if AC driven, may be particularly suitable to generate stochastic stimulation, which is expected to prevent habituation. In order to transmit the vibration of the actuator, such as solenoid valves or speakers, to the balloon with the least force, the air tubes should be as stiff and short as possible. The balloons are shielded with a silicon shield (2) that fill follow the shape of the infant's body. The shield or strap can easily be cleaned or be manufactured as a disposable. As the silicon is light but has a high friction coefficient, the strap will stay in place without applying a lot of weight on the vulnerable skin. Optionally, an additional fastening device, such as a fabric belt, may be used to fasten the strap more securely. An additional advantage of this concept is that the balloons can be used to detect breathing, as breathing might compress the balloons leading to a peak in pressure. The air tubes are preferably as much as possible in close connection with each other and/or with the silicone shield (2) in order to minimize entanglement with objects inside the incubator or with the subject.

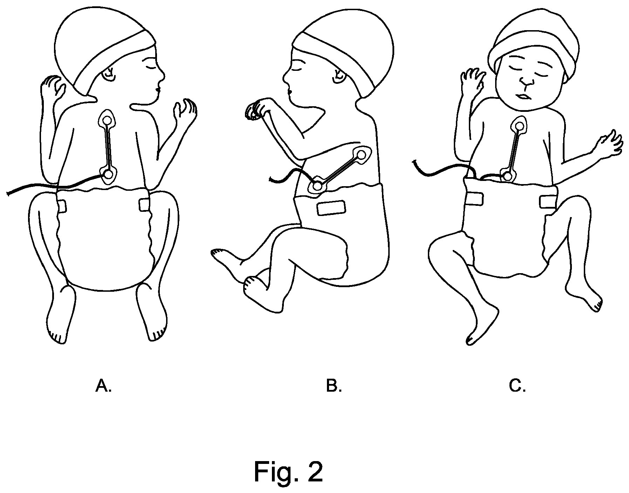

Figure 2 represents possible locations on an infant where the automated stimulation device can be placed. It can be placed on the back of the infant (A), on the side of the infant (B) or on the front of the infant (C).

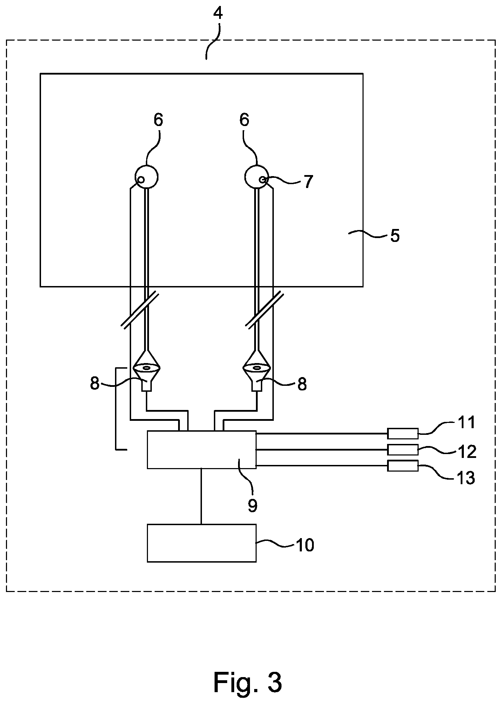

Figure 3 represents a schematic representation of the test set-up for testing the animals in the Biomedical Imaging Centre at the SPring-8 synchrotron in Japan. The stimulations were applied using a custom-made stimulation device (4). This device consists of an aluminum box (5) with two incorporated small silicon balloons (6), that are spaced roughly 5 cm from each other, with a pressure sensor (7). The balloons vibrate due to pressure changes, evoked by airtight connected speakers (8). The speakers are connected to an amplifier (9) which amplifier is connected to a computer (10) used to control the sound. The units (11), (12) and (13) are used to give signals to the balloon actuators and to read the input signal from the pressure sensor (7).

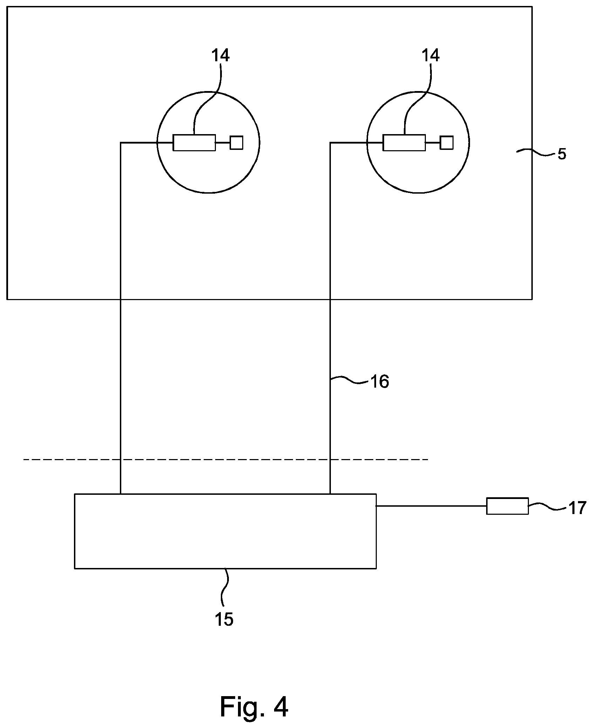

Figure 4 represents a different test set-up, for testing the effect of the stimuli. This device consists also of an aluminum box (5) with two incorporated DC motors (14) covered with a membrane as actuators. The motors are connected to a computer (15) equipped with suitable control software, and an interface cable (16). A voltage signal is given by unit (17).

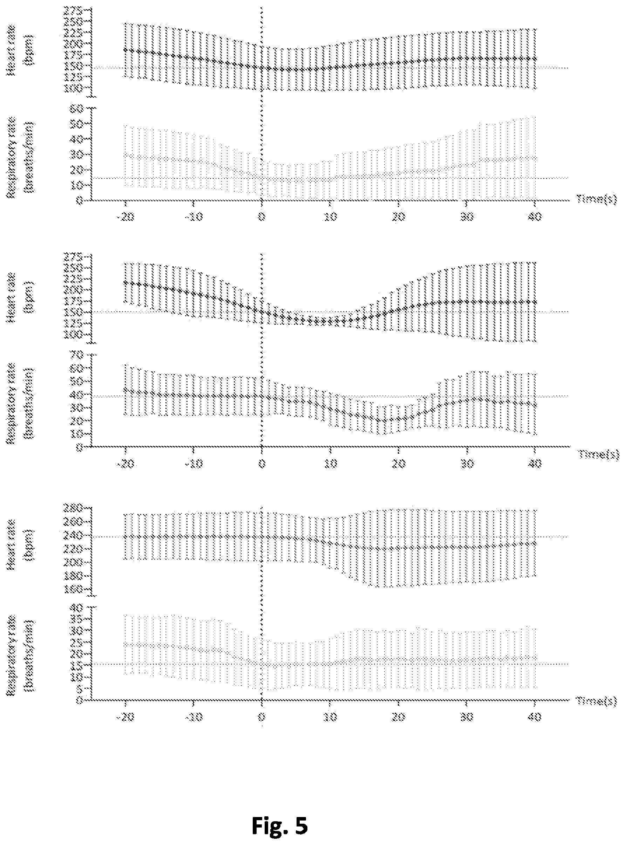

Figure 5 represents the effect of stimulation periods on heart rate and breathing rate per stimulation indicator as described in Example 3. A stimulation period was considered successful whenever it resulted in a stable or increased breathing rate and an increased heart rate within 5 seconds following the last stimulus. Stimulation started at time = 0 s, time in s is indicated on the x-axis. The top graph shows the effect of stimulation in response to unstable breathing and bradycardia. The middle graph shows the effect of stimulation in response to bradycardia. The bottom graph shows the effect of stimulation in response to unstable breathing. The top half of each graph shows the heart rate in bpm from 0 to 70 (y-axis) over time in s from -20 to 40 and the bottom half of each graph shows the respiratory rate in breaths/min from 100 to 280 (y-axis) over time in s from -20 to 40.

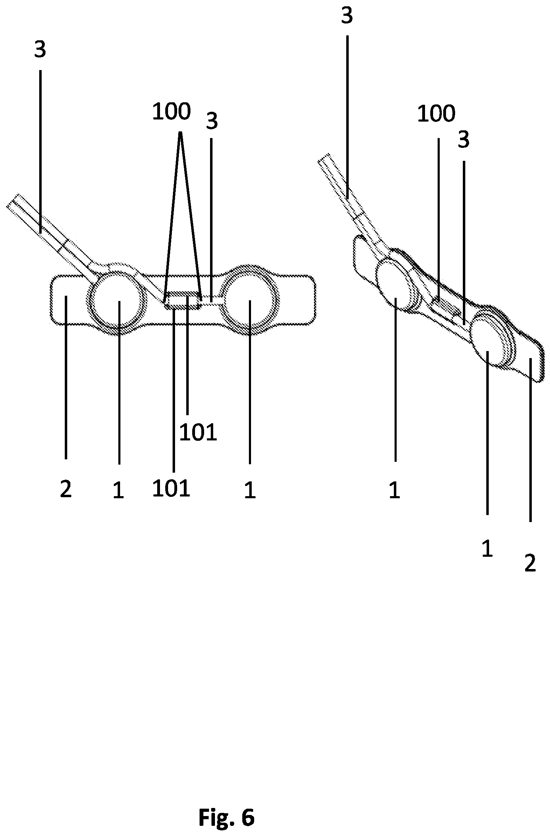

Figure 6 represents a top/frontal view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators. The stimulation device represents a similar device as represented in figure 1. Two balloons (1) that are made of medical grade silicone and are surrounded by a silicone shield (2) are airtight connected via air tubes (3) to two separate controllable solenoid valves or speakers, or otherwise devices that induce a vibration. The silicon shield will follow the shape of the body. The solenoid valves or speakers may preferably be placed outside the incubator. A holder (100) that is configured to hold securely into place one or more of the air tubes (3) may be present on the top of the device. The holder may comprise two separate walls (101) connected to the silicone shield and that are together configured to clamp an air tube. The air tubes are preferably along their entire length as much as possible in close connection with each other and/or with the silicone shield (2) in order to minimize entanglement with objects inside the incubator or with the subject and for easier handling of the stimulation device.

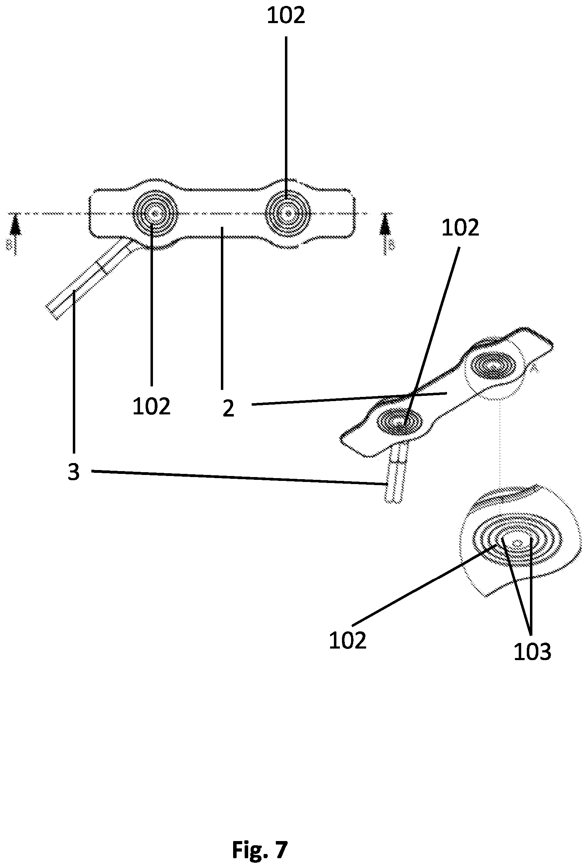

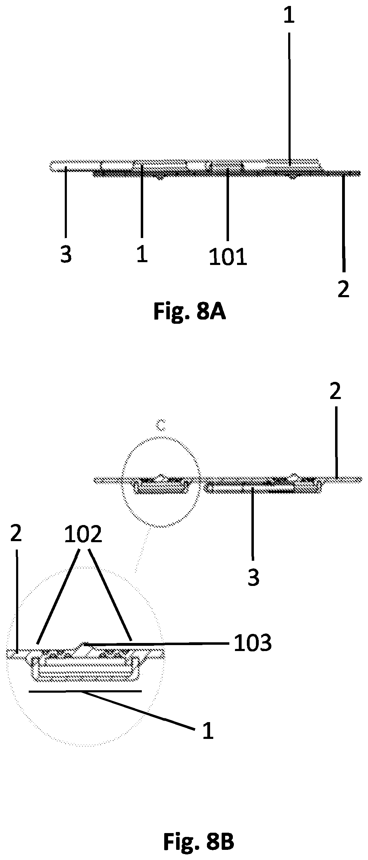

Figure 7 represents a bottom view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators. The view of the circled area A is enlarged. The stimulation device represents the same device as represented in figure 1. Two balloons (1) that are made of medical grade silicone and are surrounded by a silicone shield (2) are airtight connected via air tubes (3) to two separate controllable solenoid valves or speakers, or otherwise devices that induce a vibration. The silicon shield will follow the shape of the body. The solenoid valves or speakers may preferably be placed outside the incubator. The silicone shield that is in direct contact with the air space of each of the balloons is formed by an area (102) comprising concentric circular structures of alternating heights (a so-called wave structure), of which the central circular structure (103) preferably protrudes outward. The advantage of this area (102) is that the concentric rings that are indented in the silicone shield (2) (the wave structure) and thus configured to be not into contact with the skin of the subject are suitable for moisture transfer in order to improve the skin friendliness of the silicone material during use and to allow the silicone material to be more easily removed from the skin of the subject without causing significant irritation. An additional advantage of the wave structure of the area (102) is that the equal and reduced thickness of the wave structure, also resulting in an increased surface area of the wave structure, compared to the rest of the balloon enables an easier expansion of the wave structure towards the skin of the subject when increased pressure is applied to the balloon. Preferred dimensions of 80 mm in length and 20 mm in width are shown by double headed arrows and edge lines. These dimensions are especially suitable for the smallest (premature) infants who are usually more premature and most at risk for AoP. The dashed line B-B is used to indicate the orientation of figure 8B.

Figure 8A represents a side view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators. The stimulation device represents the same device as represented in figure 1. Two balloons (1) that are made of medical grade silicone and are surrounded by a silicone shield (2) are airtight connected via air tubes (3) to two separate controllable solenoid valves or speakers, or otherwise devices that induce a vibration. The silicon shield will follow the shape of the body. The solenoid valves or speakers may preferably be placed outside the incubator. One wall (101) of the holder (100) of an air tube is shown. Preferred dimensions of 16 mm in diameter of the balloon (1), 4 mm in total height and 1 mm in height of the silicone shield (2) are shown by double headed arrows and edge lines. These dimensions are especially suitable for the smallest (premature) infants who are usually more premature and most at risk for AoP. The height of the silicone shield (2) and the balloon (1) are at a minimum, while still enabling a functional attachment to the air tubes (3).

Figure 8B represents a cross sectional side view of an automated stimulation device for inducing a tactile inter-stimulus onset asynchrony (ISOA) effect in a subject suffering from apnoea, bradycardia and/or oxygen desaturation, the device comprising two actuators with a maximum distance between the actuators. Figure 8B illustrates a section taken along the line B-B of Figure 7. Area C is enlarged. The stimulation device represents the same device as represented in figure 1. Two balloons (1) that are made of medical grade silicone and are surrounded by a silicone shield (2) are airtight connected via air tubes (3) to two separate controllable solenoid valves or speakers, or otherwise devices that induce a vibration. The silicon shield will follow the shape of the body. The solenoid valves or speakers may preferably be placed outside the incubator. The silicone shield that is in direct contact with the air space of each of the balloons is formed by an area (102) comprising concentric circular structures of alternating heights, of which the central circular structure (103) preferably protrudes outward. The protruding central circular structure (103) advantageously provides a point of contact with the subject, whereby the transmission of vibrations via this contact is more focused and thus more effectively sensed by the subject.

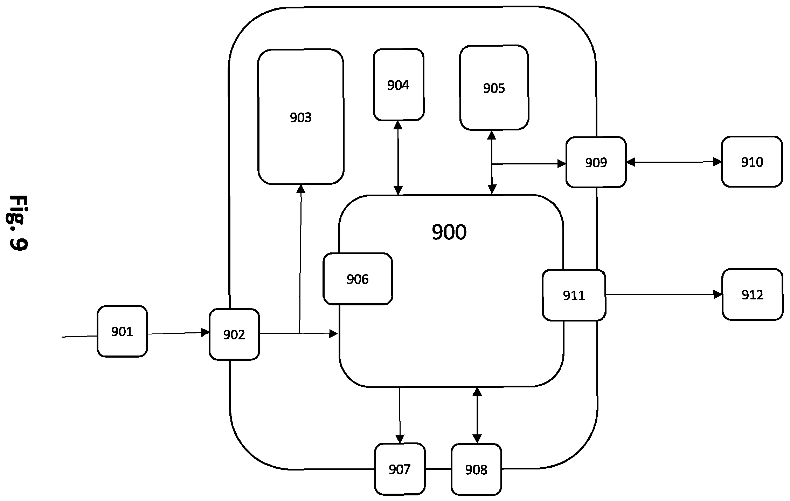

Figure 9 represents a flowchart representing a system configured to control and/or to provide feedback to an automated stimulation device for inducing a tactile inter-stimulus onset asynchrony (ISOA) effect in a subject suffering from apnoea, bradycardia and/or oxygen desaturation. The system comprises a computer (900) comprising a processor, an optional external adapter (901) for converting the voltage of a conventional outlet (e.g. 200-240 V) to low voltage (e.g. 5V), a power inlet (902), optional infrared light-emitting diodes (LEDs) (903) configured to emit infrared light directed towards the subject and/or its immediate surroundings and that can be controlled by the computer, an optional infrared camera (904) configured to detect infrared light reflected from the subject and/or its immediate surroundings which can be converted to a signal for processing by the computer. The infrared LEDs (903) and the infrared camera (904) can for example be used to monitor or detect the subject or another person interacting with the subject or the device attached to the subject. Optionally, a real-time clock (905) controlled by the computer is present. The computer is configured to receive and/or store data comprising sound data and computer-readable signals on a non-transitory computer-readable medium such as a micro SD card (906). Various readout/stimulus LEDs (907) operable by the computer can be present to indicate when a stimulus signal is sent to the actuators or they can indicate whether or not a camera or sensor attached to the device is operable or active. A stimulus can be activated or deactivated manually by a user via an ON/OFF switch (908). The computer can optionally comprise a connector (909), which is configured to connect the computer to a color sensor (910), such as an I2C color sensor that can for example be used to detect the color of a subject, which can be used to determine the oxygen level (in the blood) of the subject. Alternatively and preferably, the color sensor (910) can be used to detect a light signal generated by a monitoring or sensing system, wherein the system preferably comprises a system for detecting apnea, bradycardia, and/or hypoxia, wherein the monitoring or sensing system preferably comprises sensors for monitoring the subject's oxygen saturation, respiration and/or heart rate, preferably wherein the system comprises a pulse oximeter, respiratory monitor and/or ECG and wherein the light signal comprises a signal indicating reaching a threshold value associated with oxygen saturation, respiration and/or heart rate. The computer has a connector (911), preferably a universal serial bus (USB) connector, configured to connect the computer to the actuator device (912) via preferably a cable or less preferably via other means of connection, such as for example a wireless WiFi or Bluetooth connection. In response to a warning light signal, the color sensor (910) can thus generate a signal to be received by the computer (900), which can be used to send an activating signal by the computer (900) to the actuator device (912).

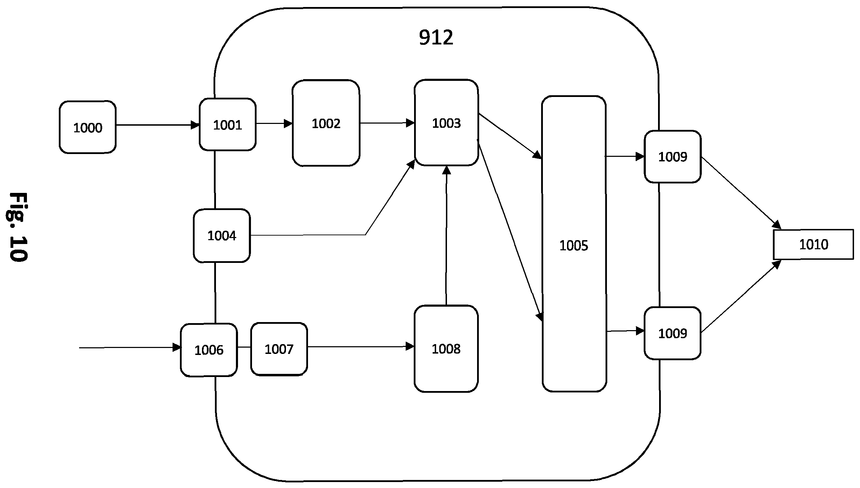

Figure 10 represents a flowchart representing an automated stimulation device for inducing a tactile inter-stimulus onset asynchrony (ISOA) effect in a subject suffering from apnoea, bradycardia and/or oxygen desaturation configured to be connected to two or more actuators. The device comprises an actuator control device (912) configured to receive an audio signal (1000), preferably via a USB cable and an inlet USB port (1001). For example, the audio signal can be generated by the system as described in figure 9. The audio signal is converted by a soundcard (1002) to a suitable signal that passes through an amplifier (1003) to activate two or more speakers (1005). The intensity of the signal passing through the amplifier (1003) can be adjusted automatically by for example the system of figure 9, or manually by a user via an external volume control (1004). The device comprises a power inlet (1006) for receiving power with a voltage of a conventional outlet (e.g. 200-240 V). Two fuses (1007) are present to provide overcurrent protection. Power is converted via a power supply (1008) into low voltage (e.g. 5V) to power the amplifier (1003). Two Luer-lock connectors that serve as outlets (1009) are each fluidly connected proximally to one of the speakers and distally to one of the actuator balloons (1010) as described in figures 1, 6, 7 and 8 via air tubes. Activated speakers (1005) can thereby thus transfer vibrations to the actuator balloons (1010) via air tubes.

The following, non-limiting examples are provided to illustrate the invention. When an ISOA with a time duration is specified in the Examples this is understood to be a Stimulus onset interval as defined by the formula (I): Stimulus onset interval (sec) = 0.31 x duration of stimulation (sec) + 0.0473 (I).

Example 1:

To evaluate how apparent tactile motion feels, a prototype was built using an Arduino Computer and two DC-motors of the following types: a coin-type that exerts vibration parallel to the skin, and a pager-type that exerts vibration perpendicular to the skin. In such vibration actuators, a small sized vibration motor is generally actuated transferring a driving force to a housing of a communication instrument thereby to vibrate the same. Vibration motors currently utilized in e.g. mobile phones are classified into coin types and elongate bar types usually employed in pagers, defined by their configurations.

The coin motors were placed on the lower arm with a piece of tape and the pager motors were placed in a small silicone shield, which was taped on the arm as well. The frequency (and thus the amplitude and strength) of the stimulation was controlled by using Pulse Width Modulation (PWM), a technique that creates a square wave by switching the signal on and off. This on-off pattern can simulate voltages in between full on (PMW 255, 5V) and off (PMW 0, 0V) by changing the amount of time the signal is on versus off. The right stimulus duration and ISOA were found in literature. The prototypes were tested on two volunteers. Duration and ISOA were increased in steps from 135ms respectively 65ms to 250ms respectively 150ms, which resulted in apparent tactile motion in all cases but with a different speed. A low PWM (pager PWM of 100 and coin PWM of 120) caused a very soft stroking feeling and a high PWM caused a sensation similar to rubbing. The apparent tactile motion was felt when the vibration motors were placed near each other, but also with a distance of 18 cm between them. The sensation felt similar for both motors, but the tingling feeling after the vibration had stopped remained longer with the coin motor as compared to the pager motor.

Example 2:

A study was conducted in experimental hutch 3 of beamline 20B2 in the Biomedical Imaging Centre at the SPring-8 synchrotron in Japan. Pups of New Zealand White rabbits were used for these experiments. All animal procedures were approved by the SPring-8 Animal Care and Monash University's Animal Ethics Committees.

Pregnant New Zealand White rabbits were anesthetized at 29 or 30 days gestational age (GA; term ≈32 d) using propofol (8mg/kg bolus, 20ml/hr intravenous infusion). An epidural was administered into the epidural space of the spinal cord (0.6 mL 2% lignocaine and 0.6 mL 0.5% bupivacaine). During the experiment anesthesia was maintained with intravenous midazolam and butorphanol (1mg/kg/hr respectively 0.5mg/kg/hr).

Pups were delivered one-by-one by caesarean section and randomly allocated into different groups for two other experimental studies. For the first study, half of the pups received an injection of saline in their trachea to increase the amount of lung liquid. The other half was gently squeezed in order to clear lung liquid via the mouth and nose. Some pups also received an intraperitoneal injection of caffeine. For the second study, pups either got an intraperitoneal injection of ibuprofen or saline. Following delivery by cutting the umbilical cord the rabbit pups had a CPAP face-mask applied and received an intraperitoneal injection naloxone (0.1mg/kg) to reverse the effect of maternally administered sedative midazolam that crosses the placenta.

The stimulations were applied using a custom-made stimulation device (Figure 3). This device comprised of an aluminum housing incorporating two small inflatable silicon balloons. The balloons were airtightly connected to speakers, which evoked a vibration in the balloons due to pressure changes when actuated. The speakers were actuated with a signal of 30 Hz with a duration of 1 sec and an ISOA of 360 msec.

The pups were placed on their side on the warmed (38°C) stimulation device, located within the experimental imaging hutch. The CPAP face-mask was connected to a purpose-build ventilator. The pups received CPAP with either 0cmH2O or 8cmH2O PEEP and an FiO2 of 0, 60 or 100%. Stimulation was started following apnea (inter breath interval of >10 sec), bradycardia (heart rate <150), or a combination of an irregular breathing pattern and a decreasing heart rate and lasted as long as considered necessary.

During the experiment airway pressures and ECG were digitally recorded (Powerlab, ADInstruments; Sydney, Australia). All animals were humanely killed at the conclusion of the experiment with an overdose of Nembutal (>100 mg/kg) administered intravenous (doe) or intraperitoneal (pups).

Stimulation was started following (a) unstable breathing (inter breath interval of >10 sec), (b) bradycardia (heart rate <150), or (c) a combination of an unstable breathing pattern and a decreased heart rate, and lasted as long as considered necessary. A stimulation period was considered successful whenever it resulted in a stable or increased breathing rate and heart rate within 5 seconds following the last stimulus.

19 pups received one or more periods of stimulation during the experiments, resulting in a total of 48 stimulation periods.

The results in in the below table 1 show that 71% (34 of 48) of the stimulation periods were successful. Table 2 shows that the success rate of the stimulation periods varied for the different stimulation indicators; 87% following bradycardia, 75% following unstable breathing and 64% following a combination of both bradycardia and unstable breathing.

Figure 5 shows the effects of the stimulation on heart rate and breathing rate per indication category.

Table 1: Experimental conditions and results per pup

| Pup | Weight | Lung liquid | Injection | PEEP | FiO2 | # stim | # succes | % |
|---|---|---|---|---|---|---|---|---|
| 1 | 36 | added | caffeine | 0 | 60 | 3 | 2 | 67 |
| 2 | 43 | added | - | 0 | 60 | 3 | 3 | 100 |
| 3 | 49 | added | - | 0 | 60 | 3 | 1 | 33 |
| 4 | 49 | added | - | 0 | 60 | 3 | 1 | 33 |
| 5 | 43 | cleared | | 8 | 60 | 1 | 0 | 0 |
| 6 | 46 | cleared | - | 0 | 60 | 5 | 5 | 100 |
| 7 | 39 | cleared | - | 0 | 60 | 3 | 2 | 67 |
| 8 | 45 | cleared | - | 8 | 60 | 1 | 1 | 100 |
| 9 | 42 | cleared | - | 8 | 60 | 2 | 2 | 100 |
| 10 | 32 | added | - | 0 | 60 | 2 | 1 | 50 |
| 11 | 29 | cleared | - | 0 | 60 | 5 | 3 | 60 |
| 12 | 36 | cleared | - | 0 | 60 | 4 | 3 | 75 |
| 13 | 37 | added | | 8 | 60 | 4 | 4 | 100 |
| 14 | 36 | cleared | | 0 | 60 | 1 | 1 | 100 |
| 15 | 26 | - | saline | 9 | 100 | 1 | 0 | 0 |
| 16 | 50 | - | saline | 0 | 50 | 2 | 1 | 50 |
| 17 | 32 | | saline | 7 | 100 | 1 | 1 | 100 |
| 18 | 31 | | saline | 9 | 100 | 2 | 1 | 50 |
| 19 | 36 | | ibu | 9 | 100 | 2 | 2 | 100 |
| Total | | | | | | 48 | 34 | 71% |

Table 2: Success rate of stimulation periods per stimulation indicator

| | | Succesful | Unsuccesful | Total |
|---|---|---|---|---|
| Unstable breathing | # stim periods | 12 | 4 | 16 |
| | % stim periods | 75% | 25% | |
| | | | | |

(continued)

|  |  | Succesful | Unsuccesful | Total |
|---|---|---|---|---|
| Bradycardia | # stim periods | 6 | 1 | 7 |
| | % stim periods | 86% | 14% | |

|  |  | Succesful | Unsuccesful | Total |
|---|---|---|---|---|
| Unstable breathing & bradycardia | # stim periods | 16 | 9 | 25 |
| | % stim periods | 64% | 36% | |

Example 3:

To evaluate how apparent tactile motion feels, a prototype was tested on 14 healthy adult volunteers using pneumatic vibration. The prototype consisted of a silicon strap with two separate air cavities, silicon tubing, a housing with two speakers and an amplifier. Stimulation was generated by playing an audio file on a personal laptop, thereby actuating the speakers, via the amplifier. An airtight connection between the speakers and the air cavities in the silicon strap made the latter to vibrate. The speakers were actuated separately in order to enable ISOA.

Three different stimulations were evaluated; stochastic stimulation of 2-30Hz moving from left to right, uniform stimulation of 8 Hz moving from right to left and a stimulation of 25 Hz moving from right to left. The duration of one stimulus was 1.2 seconds with an ISOA 0.4 seconds, resulting in a total stimulus duration of 1.6 seconds. The pause between two stimuli was set at 0.8 seconds.

The volunteers were asked to place the silicon strap on their upper leg while seated in a chair. Subsequently they received series of the three different stimulations in a random order and were asked to describe what they felt.

All 14 volunteers felt the apparent tactile motion for all three stimulations, in the right direction. However, the sensation the stimulation provoked was described different. Most volunteers (9/14) described the stimulation of 25 Hz as general vibration, while this description was only used a few times for the stochastic stimulation (4/14 volunteers) and the stimulation of 8Hz (3/14 volunteers). According to the volunteers, the slow frequency vibration gave a hopping, drumming, tapping or waving sensation. The stochastic stimulation was the hardest to describe, leading to a large variation in descriptions. It ranged from 'bumpy vibrations' to 'the sensation of a gear wheel rolling over your skin'.

**Claims**

1. An automated stimulation device for inducing a tactile inter-stimulus onset asynchrony (ISOA) effect in a subject suffering from apnea, bradycardia and/or hypoxia, the device comprising at least two actuators (1) configured for contacting a body portion of the subject, and interspaced for producing an apparent tactile movement in the subject upon sequential induction of actuation,

   wherein a duration of the actuations and an overlap in actuation time between the at least two actuators is controlled to attain an inter stimulus onset asynchrony (ISOA),
   **characterized in that** the Stimulus onset interval and duration are determined and controlled based on the following formula (I):

   $$Stimulus\ onset\ interval\ (sec) = 0.31 \times duration\ of\ stimulation\ (sec) + 0.0473\ \{I\}.$$

2. The device according to claim 1, wherein the duration of the stimulation is in the range of from 0.5 up to 5 seconds, preferably in the range of from 1 up to 3 seconds.

3. The device according to claim 1 or claim 2, wherein the actuators (1) are configured and interspaced to induce the tactile inter-stimulus onset asynchrony (ISOA) effect in the subject, wherein the subject is an infant, preterm or term, wherein preterm relates to infants born alive before 37 weeks of pregnancy are completed, and wherein the actuators are configured to be in contact with the subjects' skin, preferably wherein the subject is an infant suffering from apnea.

4. The device according to any one of claims 1 to 3, wherein the actuators are chosen from Eccentric Rotating Mass (ERM) vibration motors, linear resonant actuators (LRA), voice coils, speakers (VC), solenoids and piezoceramic elements (PCE), Electro-Active Polymers (EAP), pneumatic balloon actuators, or combinations thereof, and wherein the actuators are preferably shielded from the subject within a passive housing.

5. The device according to any one of claims 2 to 4, wherein the actuators are pneumatic inflatable actuators for providing a direct tactile stimulus to a subjects' skin, preferably pneumatic balloons.

6. The device according to any one of claims 1 to 5, wherein the device further comprises a sensing system for detecting apnea, bradycardia, and/or hypoxia.

7. The device according to any one of the previous claims, wherein the first and/or second stimulus actuation comprises a vibration with a frequency in the range of from 5 to 300 Hz, preferably of from 10 to 200 Hz, more preferably of from 10 up to 150 Hz.

8. The device according to any one of the previous claims, wherein the stimulus is provided by at least two inflatable actuators in fluid connection to two independently controllable solenoid valves or speakers, preferably further comprising a source of air pressure coupled to the valves.

9. The device according to claim 8, wherein the inflatable actuators are essentially composed of a polymeric material, preferably a medical grade silicon.

10. The device according to any one of the previous claims, comprising two or more actuators spaced at a distance of from 2 to 10 cm from each other.

11. The device according to any one of the previous claims, further comprising one or more sensors for monitoring the subject's oxygen saturation, respiration and/or heart rate, preferably wherein the device comprises a pulse oximeter, respiratory monitor and/or ECG, more preferably wherein the one or more sensors are coupled to, or integral with at least one actuator.

12. A system comprising:

i) at least one stimulation device for inducing a tactile inter-stimulus onset asynchrony (ISOA) effect in a subject suffering from apnea, bradycardia and/or hypoxia according to any one of claims 1 to 11;
ii) one or more control devices configured to control one or more characteristics of the at least one stimulation device configured to generate one or more tactile stroke sensations;
iii) one or more interfaces connecting the at least one stimulation device to the control device;
iv) one or more computing devices; and
v) a sensor system for detecting apnea, bradycardia, and/or hypoxia, the system preferably comprising one or more sensors for measuring a condition indicative of apnea, bradycardia, and/or hypoxia, preferably a pulse oximeter, respiratory monitor and/or heart rate monitor in contact with the subject;
vi) one or more control applications running on the one or more computing devices, the one or more control applications being in communication with the one or more control devices and the sensor system;

wherein the one or more control applications are configured to operate the one or more control devices, and wherein the stimulation device is configured to communicate with the one or more control systems and generate the tactile inter-stimulus onset asynchrony (ISOA) effect responsive to interaction with the one or more control devices.

13. The system according to claim 12, wherein the one or more interfaces comprise one or more housings, straps, mattresses, clamps, and/or articles of clothing.

14. The system according to claim 12 or claim 13, further comprising an incubator chamber for incubating preterm infants.

15. A computer program product comprising:
a computer readable storage medium having computer readable program code embodied thereon, the computer readable program code comprising instructions, which when executed on a computer, cause the computer to:

control one or more characteristics of the stimulation device according to any one of claims 1 to 11 for inducing a tactile inter-stimulus onset asynchrony (ISOA) effect in a subject,
to generate one or more tactile inter-stimulus onset asynchrony (ISOA) effects and to control the one or more tactile inter-stimulus onset asynchrony (ISOA) effects such that one or more tactile stroke sensations are applied to the subject when an apnea, bradycardia, and/or hypoxia condition is measured.

**Patentansprüche**

1. Automatisierte Stimulationseinrichtung zum Induzieren eines taktilen Inter-Stimulus-Onset-Asynchronie-(ISOA-) Effekts bei einem Subjekt, das an Apnoe, Bradykardie und/oder Hypoxie leidet, wobei die Einrichtung mindestens zwei Aktoren (1) umfasst, die so konfiguriert sind, dass sie einen Körperabschnitt des Subjekts berühren, und voneinander beabstandet sind, um bei sequentieller Induktion der Betätigung eine sichtbare taktile Bewegung bei dem Subjekt zu erzeugen,
wobei eine Dauer der Betätigungen und eine Überlappung der Betätigungszeit zwischen den mindestens zwei Aktoren durch eine oder mehrere Steuerungsanwendungen gesteuert wird, um eine Inter-Stimulus-Onset-Asynchronie (ISOA) zu erreichen, **dadurch gekennzeichnet, dass** das/die Stimulus-Onset-Intervall und -Dauer auf Grundlage der folgenden Formel (I) bestimmt und gesteuert werden:

$$\textit{Stimulus-Onset-Intervall (sec)} = 0{,}31 \times \textit{Dauer der Stimulation (sec)} + 0{,}0473 \ (I).$$

2. Einrichtung nach Anspruch 1, wobei die Dauer der Stimulation im Bereich von 0,5 bis zu 5 Sekunden, bevorzugt im Bereich von 1 bis zu 3 Sekunden liegt.

3. Einrichtung nach Anspruch 1 oder Anspruch 2, wobei die Aktoren (1) konfiguriert und voneinander beabstandet sind, um den taktilen Inter-Stimulus-Onset-Asynchronie-(ISOA-) Effekt bei dem Subjekt zu induzieren, wobei es sich bei dem Subjekt um einen frühgeborenen oder termingeborenen Säugling handelt, wobei sich frühgeboren auf Säuglinge bezieht, die lebendig geboren wurden, bevor 37 Wochen der Schwangerschaft abgeschlossen sind, und wobei die Aktoren konfiguriert sind, um mit der Haut des Subjekts in Berührung zu stehen, wobei es sich bei dem Subjekt bevorzugt um einen Säugling handelt, der an Apnoe leidet.

4. Einrichtung nach einem der Ansprüche 1 bis 3, wobei die Aktoren ausgewählt sind aus Vibrationsmotoren mit exzentrischer rotierender Masse (ERM), linearen Resonanzaktoren (LRA), Schwingspulen, Lautsprechern (VC), Elektromagneten und piezokeramischen Elementen (PCE), elektroaktiven Polymeren (EAP), pneumatischen Ballonaktoren oder Kombinationen davon und wobei die Aktoren bevorzugt innerhalb eines passiven Gehäuses von dem Subjekt abgeschirmt sind.

5. Einrichtung nach einem der Ansprüche 2 bis 4, wobei die Aktoren pneumatische aufblasbare Aktoren zum Bereitstellen eines direkten taktilen Stimulus an der Haut eines Subjekts sind, bevorzugt pneumatische Ballons.

6. Einrichtung nach einem der Ansprüche 1 bis 5, wobei die Einrichtung ferner ein Erfassungssystem zum Detektieren von Apnoe, Bradykardie und/oder Hypoxie umfasst.

7. Einrichtung nach einem der vorhergehenden Ansprüche, wobei die erste und/oder zweite Stimulusbetätigung eine Vibration mit einer Frequenz im Bereich von 5 bis 300 Hz, bevorzugt von 10 bis 200 Hz, besonders bevorzugt von 10 bis zu 150 Hz umfassen/umfasst.

8. Einrichtung nach einem der vorhergehenden Ansprüche, wobei der Stimulus durch mindestens zwei aufblasbare Aktoren in Fluidverbindung mit zwei unabhängig steuerbaren Elektromagnetventilen oder Lautsprechern bereitgestellt wird, bevorzugt ferner umfassend eine Luftdruckquelle, die an die Ventile gekoppelt ist.

9. Einrichtung nach Anspruch 8, wobei die aufblasbaren Aktoren im Wesentlichen aus einem Polymermaterial, bevorzugt einem Silizium medizinischer Güte, bestehen.

10. Einrichtung nach einem der vorhergehenden Ansprüche, umfassend zwei oder mehr Aktoren, die in einer Entfernung von 2 bis 10 cm zueinander beabstandet sind.

11. Einrichtung nach einem der vorhergehenden Ansprüche, ferner umfassend einen oder mehrere Sensoren zum Überwachen der Sauerstoffsättigung, der Atmung und/oder der Herzfrequenz des Subjekts, wobei die Einrichtung bevorzugt ein Pulsoxymeter, einen Atemmonitor und/oder ein EKG umfasst, wobei der eine oder die mehreren Sensoren besonders bevorzugt an mindestens einen Aktor gekoppelt oder in diesen integriert sind.

**12.** System, umfassend:

i) mindestens eine Stimulationseinrichtung zum Induzieren eines taktilen Inter-Stimulus-Onset-Asynchronie-(ISOA-)Effekts bei einem Subjekt, das an Apnoe, Bradykardie und/oder Hypoxie leidet, nach einem der Ansprüche 1 bis 11;
ii) eine oder mehrere Steuereinrichtungen, die konfiguriert sind, um eine oder mehrere Eigenschaften der mindestens einen Stimulationseinrichtung zu steuern, die konfiguriert sind, um eine oder mehrere taktile Streichelempfindungen zu erzeugen;
iii) eine oder mehrere Schnittstellen, welche die mindestens eine Stimulationseinrichtung mit der Steuereinrichtung verbinden;
iv) eine oder mehrere Recheneinrichtungen; und
v) ein Sensorsystem zum Detektieren von Apnoe, Bradykardie und/oder Hypoxie, wobei das System bevorzugt einen oder mehrere Sensoren zum Messen eines Zustands umfasst, der auf Apnoe, Bradykardie und/oder Hypoxie hinweist, bevorzugt ein Pulsoximeter, Atemmonitor und/oder Herzfrequenzmonitor in Berührung mit dem Subjekt;
vi) eine oder mehrere Steueranwendungen, die auf der einen oder den mehreren Recheneinrichtungen ausgeführt werden, wobei die eine oder mehreren Steueranwendungen in Kommunikation mit der einen oder den mehreren Steuereinrichtungen und dem Sensorsystem stehen;

wobei die eine oder mehreren Steueranwendungen konfiguriert sind, um die eine oder mehreren Steuereinrichtungen zu betreiben, und wobei die Stimulationseinrichtung konfiguriert ist, um mit dem einen oder den mehreren Steuersystemen zu kommunizieren und den taktilen Inter-Stimulus-Onset-Asynchronie-(ISOA-)Effekt als Reaktion auf eine Interaktion mit der einen oder den mehreren Steuereinrichtungen zu erzeugen.

**13.** System nach Anspruch 12, wobei die eine oder mehreren Schnittstellen ein/eine/einen oder mehrere Gehäuse, Gurte, Matratzen, Klemmen und/oder Kleidungsstücke umfassen.

**14.** System nach Anspruch 12 oder Anspruch 13, ferner umfassend eine Inkubatorkammer zum Inkubieren von frühgeborenen Säuglingen.

**15.** Computerprogrammprodukt, umfassend:
ein computerlesbares Speichermedium, auf dem computerlesbarer Programmcode ausgebildet ist, wobei der computerlesbare Programmcode Anweisungen umfasst, die, wenn sie auf einem Computer ausgeführt werden, den Computer zu Folgendem veranlassen:

Steuern einer oder mehrerer Eigenschaften der Stimulationseinrichtung nach einem der Ansprüche 1 bis 11 zum Induzieren eines taktilen Inter-Stimulus-Onset-Asynchronie-(ISOA-)Effekts bei einem Subjekt,
um einen oder mehrere taktile Inter-Stimulus-Onset-Asynchronie-(ISOA-)Effekte zu erzeugen und den einen oder die mehreren taktilen Inter-Stimulus-Onset-Asynchronie-(ISOA-)Effekte derart zu steuern, dass ein oder mehrere taktile Streichelempfindungen auf das Subjekt angewendet werden, wenn ein Apnoe-, Bradykardie- und/oder Hypoxiezustand gemessen wird.

## Revendications

**1.** Dispositif de stimulation automatique destiné à induire un effet d'asynchronisme de déclenchement entre stimuli tactiles (ISOA) chez un sujet souffrant d'apnée, de bradycardie et/ou d'hypoxie, le dispositif comprenant au moins deux actionneurs (1) conçus pour entrer en contact avec une partie du corps du sujet, et espacés pour produire un mouvement tactile apparent chez le sujet lors de l'induction séquentielle de l'actionnement,

la durée des actionnements et le chevauchement du temps d'actionnement entre lesdits au moins deux actionneurs étant commandés, de manière à atteindre un asynchronisme de déclenchement entre stimuli (ISOA),
**caractérisé en ce que** l'intervalle et la durée de déclenchement de stimulus sont déterminés et commandés sur la base de la formule suivante (I) :

*Intervalle de déclenchement de stimulus (sec) = 0,31 x durée de la stimulation (sec) + 0,0473 (I).*    (I).

2.  Dispositif selon la revendication 1, ladite durée de la stimulation étant comprise dans la plage allant de 0,5 jusqu'à 5 secondes, de préférence dans la plage allant de 1 jusqu'à 3 secondes.

3.  Dispositif selon la revendication 1 ou la revendication 2, lesdits actionneurs (1) étant conçus et espacés de manière à induire l'effet d'asynchronisme de déclenchement entre stimuli tactiles (ISOA) chez le sujet, ledit sujet étant un nourrisson, un prématuré ou un bébé né à terme, ledit prématuré se rapportant aux nourrissons nés vivants avant l'achèvement de 37 semaines de grossesse, et lesdits actionneurs étant conçus pour être en contact avec la peau des sujets, de préférence ledit sujet étant un nourrisson souffrant d'apnée.

4.  Dispositif selon l'une quelconque des revendications 1 à 3, lesdits actionneurs étant choisis parmi les moteurs à vibrations à masse rotative excentrique (ERM), les actionneurs résonants linéaires (LRA), les bobines vocales, les haut-parleurs (VC), les solénoïdes et les éléments piézocéramiques (PCE), les polymères électro-actifs (EAP), les actionneurs à ballonnet pneumatique, ou des combinaisons de ceux-ci, et lesdits actionneurs étant de préférence protégés du sujet à l'intérieur d'un boîtier passif.

5.  Dispositif selon l'une quelconque des revendications 2 à 4, lesdits actionneurs étant des actionneurs pneumatiques gonflables permettant de fournir un stimulus tactile direct à la peau d'un sujet, de préférence des ballonnets pneumatiques.

6.  Dispositif selon l'une quelconque des revendications 1 à 5, ledit dispositif comprenant en outre un système de détection permettant de détecter l'apnée, la bradycardie et/ou l'hypoxie.

7.  Dispositif selon l'une quelconque des revendications précédentes, ladite première et/ou ladite seconde activation de stimulus comprenant une vibration avec une fréquence comprise dans la plage de 5 à 300 Hz, de préférence de 10 à 200 Hz, plus préférablement de 10 à 150 Hz.

8.  Dispositif selon l'une quelconque des revendications précédentes, ledit stimulus étant fourni par au moins deux actionneurs gonflables en connexion fluidique avec deux électrovannes ou haut-parleurs commandables indépendamment, comprenant de préférence en outre une source de pression d'air couplée aux vannes.

9.  Dispositif selon la revendication 8, lesdits actionneurs gonflables étant essentiellement composés d'un matériau polymère, de préférence un silicium de qualité médicale.

10. Dispositif selon l'une quelconque des revendications précédentes, comprenant deux actionneurs ou plus espacés d'une distance de 2 à 10 cm l'un de l'autre.

11. Dispositif selon l'une quelconque des revendications précédentes, comprenant en outre un ou plusieurs capteurs destinés à surveiller la saturation en oxygène, la respiration et/ou la fréquence cardiaque du sujet, de préférence ledit dispositif comprenant un oxymètre de pouls, un moniteur respiratoire et/ou un ECG, plus préférablement lesdits un ou plusieurs capteurs étant couplés à au moins un actionneur ou faisant partie intégrante de celui-ci.

12. Système comprenant :

    i) au moins un dispositif de stimulation destiné à induire un effet d'asynchronisme de déclenchement entre stimuli tactiles (ISOA) chez un sujet souffrant d'apnée, de bradycardie et/ou d'hypoxie selon l'une quelconque des revendications 1 à 11 ;
    ii) un ou plusieurs dispositifs de commande conçus pour commander une ou plusieurs caractéristiques dudit au moins un dispositif de stimulation conçu pour générer une ou plusieurs sensations tactiles d'accident vasculaire cérébrale ;
    iii) une ou plusieurs interfaces reliant ledit au moins un dispositif de stimulation au dispositif de commande ;
    iv) un ou plusieurs dispositifs informatiques ; et
    v) un système de capteurs destiné à détecter l'apnée, la bradycardie et/ou l'hypoxie, le système comprenant de préférence un ou plusieurs capteurs destinés à mesurer un état indiquant l'apnée, la bradycardie, et/ou l'hypoxie, de préférence un oxymètre de pouls, un moniteur respiratoire et/ou un moniteur de fréquence cardiaque en contact avec le sujet ;

vi) une ou plusieurs applications de commande s'exécutant sur lesdits un ou plusieurs dispositifs informatiques, lesdites une ou plusieurs applications de commande étant en communication avec lesdits un ou plusieurs dispositifs de commande et le système de capteurs ;

lesdites une ou plusieurs applications de commande étant conçues pour faire fonctionner lesdits un ou plusieurs dispositifs de commande, et ledit dispositif de stimulation étant conçu pour communiquer avec lesdits un ou plusieurs systèmes de commande et générer l'effet d'asynchronisme de déclenchement entre stimuli tactiles (ISOA) en réponse à une interaction avec lesdits un ou plusieurs dispositifs de commande.

13. Système selon la revendication 12, lesdites une ou plusieurs interfaces comprenant un ou plusieurs logements, sangles, matelas, pinces et/ou articles vestimentaires.

14. Système selon la revendication 12 ou la revendication 13, comprenant en outre une chambre d'incubation destinée à incuber des nourrissons prématurés.

15. Produit de programme informatique, comprenant :
un support de stockage lisible par ordinateur sur lequel est incorporé un code de programme lisible par ordinateur, le code de programme lisible par ordinateur comprenant des instructions qui, lorsqu'elles sont exécutées sur un ordinateur, amènent l'ordinateur à :

commander une ou plusieurs caractéristiques du dispositif de stimulation selon l'une quelconque des revendications 1 à 11 de manière à induire un effet d'asynchronisme de déclenchement entre stimuli tactiles (ISOA) chez un sujet,
à générer un ou plusieurs effets d'asynchronisme de déclenchement entre stimuli tactiles (ISOA) et à commander lesdits un ou plusieurs effets d'asynchronisme de déclenchement entre stimuli tactiles (ISOA) de sorte qu'une ou plusieurs sensations tactiles d'accident vasculaire cérébral soient appliquées au sujet lorsqu'un état d'apnée, de bradycardie et/ou d'hypoxie est mesuré.

Fig. 1

A.          B.          C.

Fig. 2

Fig. 3

Fig. 4

**Fig. 5**

**Fig. 6**

**Fig. 7**

**Fig. 8A**

**Fig. 8B**

**Fig. 9**

**Fig. 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8517981 B
- US 2018160967 A1
- WO 2019036408 A1
- US 2010318007 A1
- WO 2015175673 A1